# FASCICULE DE BREVET EUROPEEN

(11) **EP 4 338 724 B1**
(45) Date de publication et mention de la délivrance du brevet: **07.05.2025**
(21) Numéro de dépôt: 22196220.2
(22) Date de dépôt: 16.09.2022
(51) Int. Cl.: A61K 8/49, A61K 8/60, A61K 8/9789, A61Q 19/00, A61Q 19/08

(54) **EXTRAIT ALCOOLIQUE D ECORCES DE CITRUS DEPRESSA, SON PROCEDE D OBTENTION, ET COMPOSITION COSMETIQUE OU DERMATOLOGIQUE LE CONTENANT**
ALKOHOLISCHER AUSZUG AUS DEN SCHALEN VON CITRUS DEPRESSA, SEIN HERSTELLUNGSVERFAHREN UND KOSMETISCHE ODER DERMATOLOGISCHE ZUSAMMENSETZUNG, DIE IHN ENTHÄLT
ALCOHOLIC EXTRACT OF CITRUS DEPRESSA BARK, METHOD FOR OBTAINING SAME, AND COSMETIC OR DERMATOLOGICAL COMPOSITION CONTAINING SAME

(43) Date de publication de la demande: 20.03.2024
(73) Titulaire: Chanel Parfums Beauté, 92200 Neuilly-sur-Seine (FR)
(72) Inventeur: RILHAC, Vincent, 93694 PANTIN CEDEX (FR); GILLET, MAEVA, 93694 PANTIN CEDEX (FR)
(74) Mandataire: Plasseraud IP

(56) Documents cités:
- KR-A- 20120 043 288
- KR-A- 20170 121 468
- DATABASE GNPD [online] MINTEL; 30 August 2008 (2008-08-30), ANONYMOUS: "Seasonal Essence '08-AW", XP055921494, retrieved from https://www.gnpd.com/sinatra/recordpage/968901/ Database accession no. 968901

## Description

L'invention concerne un extrait d'écorces de *Citrus depressa,* son procédé d'obtention, une composition cosmétique ou dermatologique le contenant, ainsi que son utilisation cosmétique.

La peau est principalement constituée de trois couches, à savoir, en partant de la plus superficielle, l'épiderme, le derme et l'hypoderme.

La couche externe de la peau, l'épiderme, est stratifiée et contribue largement à assurer la protection de la peau vis-à-vis des agressions extérieures. Il est composé principalement de trois types de cellules qui sont les kératinocytes, très majoritaires, les mélanocytes et les cellules de Langerhans. Chacun de ces types cellulaires contribue par ses fonctions propres au rôle essentiel joué dans l'organisme par la peau, notamment le rôle de protection de l'organisme des agressions extérieures (climat, rayons ultraviolets, tabac...), appelé «fonction barrière».

Le vieillissement de l'épiderme se manifeste principalement par la réduction de son épaisseur. L'atrophie de l'épiderme est la conséquence du ralentissement de la prolifération des kératinocytes et de l'accumulation de kératinocytes sénescents. La couche cornée devient terne.

La desquamation est un phénomène naturel lié au fait que l'épiderme, qui constitue la couche supérieure de la peau, est en constant renouvellement. L'épiderme est constitué de plusieurs assises de cellules, dont la plus profonde est l'assise basale constituée de cellules indifférenciées. Au cours du temps, ces cellules vont se différencier et migrer vers la surface de l'épiderme en constituant les différentes assises de celui-ci, jusqu'à former à la surface de l'épiderme les cornéocytes qui sont des cellules mortes qui s'éliminent par desquamation. Cette perte en surface est compensée par la migration de cellules de l'assise basale vers la surface de l'épiderme. Il s'agit du renouvellement perpétuel de la peau.

Toutefois, une desquamation trop importante ou irrégulière des cellules du stratum corneum peut conduire à la formation d'amas de cellules de grandes tailles, épais, visibles à l'œil nu, et dénommés «squames», ou «pellicules» dans le cadre du cuir chevelu, ou dans d'autres situations, à un amincissement du stratum corneum générant un sensation de tiraillement et d'inconfort. Les troubles de la desquamation, résultant d'une desquamation anormale ou irrégulière, peuvent aboutir à une fragilité, voire un défaut des propriétés barrières de l'épiderme.

La présence de squames ou les états pelliculaires peuvent être des états fréquents, récidivants, et présentent un caractère inesthétique et un inconfort manifeste.

Il existe donc un besoin de disposer de nouveaux actifs agissant sur la desquamation excessive de la peau ou du cuir chevelu pour la limiter et/ou la prévenir, de manière à contribuer au confort cutané.

Le *Citrus depressa* est un agrume de la famille des Rutaceae, cultivé au Japon. Les extraits, généralement aqueux, de ces fruits peuvent être recherchés pour être utilisés en cosmétique.

Le document KR 2012/0043288 propose par exemple l'utilisation d'un extrait de *Citrus depressa* comme agent blanchissant, stimulant le renouvellement de la peau.

KR20170121468 A divulgue une composition comprenant un extrait d'écorces de Citrus, dans laquelle l'extrait d'écorces de Citrus comprend nobilétine et tangérétin, et son utilisation pour regénérer la peau

Mintel GNPD Record ID 968901 " Seasonal Essence '08-AW", Aug 2008, divulgue une composition pour contribuer au confort cutané comprenant un extrait d'écorces de Citrus depressa.

Les auteurs de la présente invention ont maintenant mis en évidence, de façon tout à fait surprenante, qu'un extrait alcoolique d'écorces de *Citrus depressa,* présentait une activité sur les mécanismes impliqués dans la desquamation, en particulier par l'inhibition de certaines enzymes impliquées dans la desquamation.

Cette constatation a conduit à la mise au point de nouvelles compositions cosmétiques non thérapeutiques, plus particulièrement utiles pour toutes les applications dans lesquelles on cherche à améliorer le confort cutané en prévenant ou limitant la desquamation.

L'invention concerne donc, selon un premier aspect, un extrait alcoolique d'écorces de *Citrus depressa,* comprenant un mélange d'hespéridine, de nobilétine et d'isosinensétine.

L'invention a également pour objet une composition cosmétique comprenant, dans un milieu physiologiquement acceptable, au moins un extrait alcoolique d'écorces de *Citrus depressa* selon l'invention.

L'invention concerne, selon un troisième aspect, un procédé d'extraction d'écorces de *Citrus depressa,* comprenant les étapes suivantes :
a) extraction des écorces de *Citrus depressa,* préalablement séchées et broyées, avec au moins un solvant alcoolique ;
b) filtration du mélange issu de l'étape a) et optionnellement, décoloration du filtrat obtenu par adsorption sur charbon actif,
c) concentration de l'extrait sec par évaporation jusqu'à une teneur en extrait sec comprise entre 5 et 20%, de préférence entre 9 et 12%,
d) décantation du mélange obtenu en c) pendant au moins 6h jusqu'à obtenir 4 phases distinctes;
e) séparation des phases formées à l'étape d) par élimination des deux phases inférieures solide et huileuse, et de la phase supérieure d'huile essentielle, et récupération de la phase alcoolique comprenant l'extrait d'écorces de *Citrus depressa.*

Enfin, l'invention a pour objet, selon un quatrième aspect, l'utilisation cosmétique non thérapeutique d'un extrait alcoolique d'écorces de *Citrus depressa* tel que décrit précédemment, pour limiter/protéger de la desquamation excessive et contribuer au confort cutané.

### Brève description des figures

La **Figure** illustre la diminution de l'expression génique des kallikréines (KLK) KLK5 et KLK7, deux protéases à serine impliquées dans la desquamation cutanée après traitement avec extrait alcoolique d'écorces de *Citrus depressa* selon l'invention à 0,033%.

### Citrus depressa

Le *Citrus depressa,* aussi connu sous le nom de *shi̅kwa̅sa̅,* est un agrume de la famille des Rutaceae, cultivé au Japon.

Le mot *shi̅kwa̅sa̅* désigne en français la plante et le fruit.

C'est un petit arbre buissonnant d'environ 5 m., vigoureux et dense, fructifère. La floraison blanche a lieu en avril (diamètre de la fleur 3 cm). Le fruit se récolte d'octobre à novembre.

Le fruit est petit (diamètre 2,5 à 4 cm, hauteur 2 à 3 cm, poids de 25 à 60 g), déprimé aux pôles, à péricarpe (écorce) fin. Il contient 8 ou 10 quartiers spécialement juteux et des graines. La pulpe est molle et gélatineuse et le jus acide. L'essence contenue dans le péricarpe a un parfum de mandarine satsuma. Le jus est ainsi principalement utilisé dans l'industrie alimentaire, et l'écorce pour la préparation d'huiles essentielles.

L'extrait selon l'invention est obtenu à partir d'écorces de *Citrus depressa,* et de préférence, obtenues après pressage du fruit pour obtenir le jus.

### Extrait alcoolique d'écorces de Citrus depressa

L'extrait alcoolique d'écorces de *Citrus depressa,* selon l'invention comprend un mélange d'hespéridine, de nobilétine et d'isosinensétine.

La combinaison de ces trois molécules d'intérêt, obtenues au moyen d'un procédé d'extraction bien spécifique, permet en effet la diminution de l'expression génique des kallikréines (KLK) KLK5 et KLK7, deux protéases à serine impliquées dans la desquamation cutanée, permettant ainsi limiter/protéger de la desquamation excessive et contribuer au confort cutané.

Selon un mode préféré de réalisation, l'extrait alcoolique d'écorces de *Citrus depressa* selon l'invention comprend :
- 0.1 à 5% en poids, par rapport au poids d'extrait sec, d'hespéridine, de préférence de 0.5 à 2% en poids,
- 0.1 à 5% en poids, de nobilétine, de préférence de 1 à 2% en poids,
- 0.1 à 3% en poids, d'isosinensétine, de préférence de 0.5 à 1% en poids,

Les pourcentages étant exprimés en poids, par rapport au poids d'extrait sec.

De préférence, l'hespéridine, la nobilétine et l'isosinensétine sont présent en un rapport pondéral hespéridine/nobilétine/isosinensétine compris entre 0.5/1/1.5 et 1.5/3/4 et de préférence, est de 1.1/1.4/0.7.

### Procédé de préparation d'un extrait alcoolique d'écorces de Citrus depressa

Selon un mode de réalisation particulier, l'invention a pour objet un procédé d'extraction d'écorces de *Citrus depressa,* comprenant les étapes suivantes :
a) extraction des écorces de *Citrus depressa,* préalablement séchées et broyées, avec au moins un solvant alcoolique ;
b) filtration du mélange issu de l'étape a) et optionnellement, décoloration du filtrat obtenu par adsorption sur charbon actif,
c) concentration de l'extrait sec par évaporation jusqu'à une teneur en extrait sec comprise entre 5 et 20%, de préférence entre 9 et 12%,
d) décantation du mélange obtenu en c) pendant au moins 6h jusqu'à obtenir 4 phases distinctes;
e) séparation des phases formées à l'étape d) par élimination des deux phases inférieures solide et huileuse, et de la phase supérieure d'huile essentielle, et récupération de la phase alcoolique comprenant l'extrait d'écorces de *Citrus depressa.*

En effet, il est du mérite de la demanderesse d'avoir mis en évidence qu'en procédant à l'extraction d'écorces de *Citrus depressa* dans des conditions bien spécifiques, il était possible d'obtenir un extrait enrichi en molécules d'intérêt pour prévenir la desquamation, telles que l'hespéridine, la nobilétine et l'isosinensétine.

Le procédé selon l'invention met en œuvre une première étape a) d'extraction des écorces de *Citrus depressa* avec au moins un solvant alcoolique.

Les écorces issues du pressage du fruit sont de préférence séchées puis réduites en poudre dispersible par tout procédé de broyage classiquement connu de l'homme du métier, par exemple à température ambiante dans un broyeur à couteaux ou, selon un mode de réalisation préféré, par broyage à basse température.

De préférence, la poudre dispersible d'écorces de *Citrus depressa* mise en œuvre pour la préparation de l'extrait selon l'invention présente une taille moyenne de particules inférieure à 500 µm, préférentiellement inférieure à 300 µm.

Dans l'étape a), les écorces de *Citrus depressa* sont soumises à une extraction par un ou plusieurs solvants alcooliques, par exemple choisis parmi :
- les monoalcools en C₁-C₄, tels que par exemple le méthanol, l'éthanol ou l'isopropanol ; et
- les diols, tels que par exemple le propylène glycol, le 1,3-propanediol ou le dipropylène glycol.

De préférence, le solvant alcoolique est un monoalcool comprenant de 2 à 4 atomes de carbone, plus préférentiellement l'éthanol.

L'extraction est généralement réalisée en immergeant sous agitation les écorces de *Citrus depressa* dans un ou plusieurs des solvants cités ci-dessus à des températures allant, par exemple, de la température ambiante à 80°C, pendant une durée d'environ 30 minutes à 8 h. De préférence, l'extraction de l'étape a) est réalisée pendant une durée comprise entre 1 et 5 heures, à une température comprise entre 50°C et 70°C.

En particulier, le rapport pondéral d'écorces de *Citrus depressa*/*solvant* alcoolique compris entre 1/1 et 1/15, et de préférence est de 1/8.

L'étape d'extraction est de préférence suivie d'un tamisage entre 50 µm et 150µm, de préférence à 100µm.

Selon un mode particulier de réalisation, l'étape a) d'extraction est réalisée deux fois. Le procédé de l'invention comprend alors :
a) une première extraction des écorces de *Citrus depressa,* préalablement séchées et broyées, avec un solvant alcoolique, de préférence l'éthanol, à une température comprise entre 50°C et 70°C pendant 1h à 5h, puis tamisage entre 50 µm et 150µm;
a') une seconde extraction des drèches d'écorces de *Citrus depressa* obtenues à l'étape a), avec un solvant alcoolique, de préférence l'éthanol, à une température comprise entre 50°C et 70°C pendant 1h à 5h , puis tamisage entre 50 µm et 150µm.

A l'issue de l'étape a), le procédé selon l'invention met en œuvre une l'étape b) de filtration opérée de préférence jusqu'à un seuil de 30 µm, de préférence de 15 µm.

L'étape b) comprend en outre optionnellement décoloration du filtrat obtenu par adsorption des pigments sur charbon actif. La décoloration du filtrat sur charbon actif est par exemple opérée pendant 1 à 6h, de préférence pendant 3h, sous agitation, à une température comprise entre 20 et 30°C, de préférence à température ambiante (20°C).

Pour la décoloration, la teneur en charbon actif est généralement comprise entre 20 et 40% en poids, par rapport au poids total de la matière sèche de l'extrait, de préférence de l'ordre de 30% en poids.

A l'issue de la décoloration, le charbon actif est éliminé par filtration jusqu'à un seuil de 5 µm, de préférence de 2µm.

Le filtrat alcoolique issu de l'étape b) éventuellement décoloré, est ensuite concentré par évaporation du solvant jusqu'à obtenir une teneur en extrait sec comprise entre 5 et 20%, de préférence entre 9 et 12% (étape c)). L'évaporation du solvant peut par exemple être effectuée au moyen d'un évaporateur rotatif ou d'un évaporateur à flux tombant.

L'extrait concentré issu de l'étape c) est ensuite laissé à décanter pendant au moins 6h jusqu'à obtenir 4 phases distinctes : deux phases inférieures solide et huileuse, une phase supérieure d'huile essentielle, et une phase alcoolique intermédiaire comprenant l'extrait d'écorces de *Citrus depressa.* La décantation peut par exemple se faire en ampoule.

Selon un mode préféré de réalisation, l'étape d) de décantation est réalisée pendant au moins 12h, de préférence au moins 24h, et plus préférentiellement pendant 24 à 48h.

La décantation est conduite à une température comprise entre 0 et 25°C, de préférence entre 1 et 10°C, plus préférentiellement entre 4 et 5°C.

Une fois la décantation terminée et les 4 phases obtenues, la phase d'intérêt est extraite par élimination des deux phases inférieures solide et huileuse, et de la phase supérieure contenant les huiles essentielles, et récupération de la phase alcoolique comprenant l'extrait d'écorces de *Citrus depressa.*

L'extrait alcoolique d'écorces de *Citrus depressa* peut ensuite être filtré (étape f)) jusqu'à un seuil de 5 µm, de préférence de 2µm, puis dilué dans un solvant alcoolique différent du solvant d'extraction mis en œuvre à l'étape a).

Le solvant d'extraction introduit à l'étape a) est ensuite évaporé jusqu'à atteindre une concentration résiduelle en solvant d'extraction inférieure à 1%, de préférence inférieure à 0, 5% (étape g)).

Enfin, le mélange obtenu à l'issue de l'étape g) est laissé à décanter pendant au moins 6h, de préférence 12h, puis filtré jusqu'à un seuil de 5 µm, de préférence de 2µm. La décantation est conduite, comme précédemment, à une température comprise entre 0 et 25°C, de préférence entre 1 et 10°C, plus préférentiellement entre 4 et 5°C.

Ainsi, selon un mode préféré de réalisation, le procédé d'extraction d'écorces de *Citrus depressa,* comprenant les étapes suivantes :
a) une première extraction des écorces de *Citrus depressa,* préalablement séchées et broyées, avec un solvant alcoolique, de préférence l'éthanol, à une température comprise entre 50°C et 70°C pendant 1h à 5h, puis tamisage entre 50 µm et 150µm;
a') une seconde extraction des drèches d'écorces de *Citrus depressa* obtenues à l'étape a), avec un solvant alcoolique, de préférence l'éthanol, à une température comprise entre 50°C et 70°C pendant 1h à 5h , puis tamisage entre 50 µm et 150µm.
b) filtration du mélange issu de l'étape a) et optionnellement, décoloration du filtrat obtenu par adsorption sur charbon actif,
c) concentration de l'extrait sec par évaporation jusqu'à une teneur en extrait sec comprise entre 5 et 20%, de préférence entre 9 et 12%,
d) décantation du mélange obtenu en c) pendant au moins 6h jusqu'à obtenir 4 phases distinctes;
e) séparation des phases formées à l'étape d) par élimination des deux phases inférieures solide et huileuse, et de la phase supérieure d'huile essentielle, et récupération de la phase alcoolique comprenant l'extrait d'écorces de *Citrus depressa,*
f) filtration de la fraction alcoolique récupérée à l'étape e) jusqu'à un seuil de 5 µm, de préférence de 2µm puis dilution finale de l'extrait filtré dans un autre solvant alcoolique,
g) évaporation du solvant d'extraction introduit à l'étape a) jusqu'à atteindre une concentration résiduelle en solvant d'extraction inférieure à 1%, de préférence inférieure à 0,5%
h) décantation du mélange obtenu en g) pendant au moins 6h puis filtration jusqu'à un seuil de 5 µm, de préférence de 2µm.

Plus préférentiellement encore, le procédé d'extraction d'écorces de *Citrus depressa,* comprenant les étapes suivantes :
a) une première extraction des écorces de *Citrus depressa,* préalablement séchées et broyées, avec de l'éthanol, dans un rapport pondéral d'écorces de *Citrus depressa*/*éthanol* de 1/8, à une température comprise entre 50°C et 70°C pendant 1h à 5h, puis tamisage à 100 µm;
a') une seconde extraction des drèches d'écorces de *Citrus depressa* obtenues à l'étape a), avec de l'éthanol, dans un rapport pondéral d'écorces de *Citrus depressa*/*éthanol* de 1/8, à une température comprise entre 50°C et 70°C pendant 1h à 5h , puis tamisage à 100 µm.
b) filtration du mélange issu de l'étape a) et la décoloration du filtrat obtenu par adsorption sur charbon actif pendant 3h à température ambiante,
c) concentration de l'extrait sec par évaporation jusqu'à une teneur en extrait sec comprise entre 10 et 11%,
d) décantation du mélange obtenu en c) pendant au moins 12h, à une température comprise entre 4 et 5°C, jusqu'à obtenir 4 phases distinctes ;
e) séparation des 4 phases formées à l'étape d) par élimination des deux phases inférieures solide et huileuse, et de la phase supérieure d'huile essentielle, et récupération de la phase éthanolique comprenant l'extrait d'écorces de *Citrus depressa,*
f) filtration de la fraction éthanolique récupérée à l'étape e) jusqu'à un seuil de 2µm puis dilution finale de l'extrait filtré dans du pentylène glycol,
g) évaporation de l'éthanol d'extraction introduit à l'étape a) jusqu'à atteindre une concentration résiduelle en éthanol inférieure à 0,5%
h) décantation du mélange obtenu en g) pendant au moins 12h, à une température comprise entre 4 et 5°C puis filtration jusqu'à un seuil de 2µm.

### Composition cosmétique

La présente invention a encore pour objet une composition cosmétique comprenant, dans un milieu physiologiquement acceptable, au moins un extrait alcoolique extrait d'écorces de *Citrus depressa* tel que décrit précédemment.

La composition mise en œuvre selon l'invention comprend généralement, outre l'extrait décrit précédemment, un milieu physiologiquement acceptable et de préférence cosmétiquement acceptable, c'est-à-dire qui convient à une utilisation en contact avec la peau humaine sans risque de toxicité, d'incompatibilité, d'instabilité, de réponse allergique et notamment qui ne provoque pas de sensations d'inconfort (rougeurs, tiraillements, picotements).

Avantageusement, ladite composition cosmétique ou dermatologique peut se présenter sous la forme d'une poudre, d'une émulsion, d'une microémulsion, d'une nanoémulsion, d'une suspension, d'une solution d'une lotion, d'une crème, d'un gel aqueux ou hydroalcoolique, d'une mousse, d'un sérum, d'une solution ou d'une dispersion pour aérosol, ou d'une dispersion de vésicules lipidiques.

Dans le cas d'une émulsion, il peut s'agir d'une émulsion eau-dans-huile ou huile-dans-eau.

La composition cosmétique ou dermatologique selon l'invention peut comprendre également un solvant choisi en fonction des différents ingrédients et de la forme d'administration.

A titre d'exemples, on peut citer l'eau (de préférence de l'eau déminéralisée ou des eaux florales), un alcool tel que l'éthanol.

Ladite composition cosmétique peut également comprendre, en outre de l'extrait selon l'invention, au moins un additif usuel dans le domaine, tel que par exemple au moins un composé choisi parmi un agent émollient ou humectant, un agent gélifiant et/ou épaississant, un agent tensioactif, une huile, un agent actif, un colorant, un conservateur, un agent antioxydant, un agent actif, une poudre organique ou inorganique, un filtre solaire et un parfum, et notamment :
- un ou plusieurs agent(s) humectant(s), tels que les polyols (glycérine, diglycérine, le propylène glycol, le caprylyl glycol, le pentylène glycol, l'hexanediol), les sucres, les glycosaminoglycanes tels que l'acide hyaluronique et ses sels et esters; et les polyquaterniums tel que le lipidure PMB. Ledit agent humectant sera présent dans la composition à une teneur de l'ordre de 0 à 30%, de préférence 0.005 à 10% en poids total de la composition.
- Un ou plusieurs agent(s) émollient(s) qui peuvent être choisi(s) par exemple parmi les esters tels que les esters de jojoba, les esters d'acides gras et d'alcool gras (octyldodecyl myristate, triethylhexanoin, Dicaprylyl carbonate, Isostearyl isostearate, caprylic/capric triglyceride), les beurres tels que les beurres de karité (butyrospernum parkii butter extract, shea butter ethyl esters, commercialisés sous les noms de LIPEX SHEASOFT, LIPEX SHEA-U, LIPEX SHEA, LIPEX SHEALIGHT, LIPEX SHEA TRIS) ou de moringa (moringa oil/hydrogenated moringa oil esters), les cires (acacia decurrens flower wax & helianthus annuus cera seed seed wax, C10-18 triglycerides) , les huiles végétales, le phytosqualane, les alcanes (undecane, tridecane). Ledit agent émollient sera présent dans la composition à une teneur de l'ordre de 0,1 à 30%, de préférence 0,5 à 10% en poids total de la composition.
- Un ou plusieurs agent(s) gélifiants(s) et/ou épaississant(s) de la phase aqueuse, choisi par exemple parmi les dérivés cellulosiques, gommes d'origine végétale (guar, caroube, alginates, carraghenanes, pectines), d'origine microbienne (xanthane), les argiles (laponite), les homo- et copolymères réticulés ou non, hydrophiles ou amphiphiles, d'acide acryloylméthylpropane sulfonique (AMPS) et/ou d'acrylamide et/ou d'acide acrylique et/ou de sels ou d'esters d'acide acrylique (commercialisés sous les noms ARISTOFLEX AVC, Aristoflex AVS, Aristoflex HMB, SIMULGEL NS, Simulgel EG, Simulgel 600, Simulgel 800, Pemulen, carbopol, Sepiplus 400, Seppimax zen, Sepiplus S, COSMEDIA SP). Ledit agent gélifiant et/ou épaississant sera présent dans la composition à une teneur de l'ordre de 0,1 à 10% en poids total de la composition.
- Un ou plusieurs agent(s) tensioactif(s), notamment :
   *les tensioactifs anioniques tels que les isethionates, les taurates, les sarcosinates, les glycinates, les glutamates, les phosphates (C20-22 alkyl phosphate commercialisé sous le nom SENSANOV WR)
   * les tensioactifs amphotères tel que les dérivés de la bétaine, les amphoacétates
   * les tensioactifs non ioniques tel que les dérivés de polyglycérol, les dérivés de sucre (les dérivés de glucosides ou de xylosides commercialisés sous le nom MONTANOV 68, MONTANOV 202, Montanov 82, MONTANOV L, EASYNOV), les lecithines.

Ledit agent tensioactif, sera présent dans une teneur de l'ordre de 0.1 à 15%, de préférence 0,5 à 10% en poids, par rapport au poids total de la composition ;
- Un ou plusieurs agent (s) actif(s) d'origine naturelle, biotechnologique ou synthétique ayant une activité biologique et ayant une efficacité sur la peau via des sites biologiques, par exemple choisi parmi les vitamines tels que la vitamine C et ses dérivés (ascorbyl glucoside, 3-o-ethyl ascorbic acid, le tétraisopalmitate d'ascorbyle) ,la vitamine A et ses dérivés, la vitamine E et ses dérivés, la vitamine B3 ou Niacinamide, le panthénol, les oligo éléments, l'allantoïne, l'adenosine, les peptides (Palmitoyl tetrapeptide-7, Palmitoyl Tripeptide-1, Palmitoyl Pentapeptide-4, Acetyl Dipeptide-1 Cetyl Ester, Acetyl Tetrapeptide-5 commercialisés sous le nom NP RIGIN, MATRIXYL 3000, IDEALIFT, EYESERYL), les extraits végétaux (glycyrrhiza glabra extract, centella asiatica leaf extract, secale cereale seed extract), les extraits de levures, les alpha hydroxyacides tels que l'acide glycolique ou lactique, l'acide tranexamique et ses dérivés tel que le cetyl tranexamique ester etc. Ledit agent actif sera présent dans la composition à une teneur de l'ordre de 0,1 à 10% en poids total de la composition.

D'autres additifs habituellement utilisés en cosmétique peuvent également être présents dans la composition selon l'invention, notamment des conservateurs, des agents antioxydants ou des parfums bien connus dans le domaine technique.

L'homme du métier est en mesure de choisir, parmi l'ensemble de ces éventuels additifs, aussi bien la nature que la quantité de ceux qui seront ajoutés à la composition, de telle sorte que celle-ci conserve l'ensemble de ses propriétés.

L'invention a également pour objet l'utilisation cosmétique non thérapeutique de l'extrait alcoolique d'écorces de *Citrus depressa* pour limiter/protéger de la desquamation excessive et contribuer au confort cutané.

Dans ce mode de réalisation, l'extrait ou la composition est appliqué sur une peau altérée mais non pathologique.

L'invention a encore pour objet l'utilisation cosmétique non thérapeutique d'un extrait alcoolique d'écorces de *Citrus depressa* tel que décrit précédemment, en tant qu'un agent inhibiteur de l'expression génique des kallikréines (KLK) KLK5 et KLK7.

L'invention sera maintenant illustrée par les exemples non limitatifs suivants.

### Exemple 1 : Inhibition de l'expression d'enzymes impliquées dans la desquamation dans des kératinocytes humains normaux traités avec l'extrait alcoolique d'écorces de Citrus depressa selon l'invention

### Préparation de l'extrait :

Un extrait alcoolique d'écorces de *Citrus depressa* selon l'invention a été préparé, selon les étapes suivantes :
a) 1kg d'écorces séchées sont broyées en poudre. Les écorces sont extraites dans 8kg d'éthanol à 96° sous agitation à 60°C pendant 2h. Le mélange est tamisé à 100µm pour récupérer les drèches et mettre l'extrait de coté.
a')Les drèches sont à nouveau extraites dans 8kg d'éthanol à 96° sous agitation à 60°C pendant 2h. Le mélange est tamisé à 100µm.
b) Les fractions liquides issues des étapes a) et a') sont rassemblées puis filtrées à 15µm.
   La fraction éthanolique est décolorée avec 70g de charbon actifs (30% en masse par rapport à la matière sèche d'extrait) sous agitation pendant 3h à température ambiante
   Le charbon actif est ensuite éliminé par filtration à 2µm
c) L'extrait éthanolique est concentré par évaporation de l'éthanol à l'évaporateur rotatif ou à l'évaporateur à flux tombant pour obtenir un extrait concentré avec une matière sèche comprise en 10% et 11%
d) L'ensemble est mis à décanter à 4°C en ampoule pendant 1 à 2 jours
e) Les phases inférieures solides et huileuse sont éliminées ainsi que la phase supérieure contenant des huiles essentielles
f) La fraction éthanolique est récupérée et filtré à 2µm. La mesure de matière sèche est effectuée sur l'extrait et contient 135g d'extrait sec.
   1215g de pentylène glycol sont ajouté à l'extrait éthanolique pour diluer l'extrait final à 10% de matière sèche
g) L'éthanol est évaporé complétement à l'évaporateur rotatif ou à l'évaporateur à flux tombant (concentration finale en éthanol inférieure à 0.5%)
h) L'extrait est laissé une nuit à 4°C et filtré à 2µm. 1350g d'extrait final 1701EXT sont obtenus

### Protocole :

Des kératinocytes épidermiques humains normaux issus de trois donneurs différents ont été ensemencés en plaque 24 puits et cultivés en milieu complémenté kératinocyte-SFM (k-SFM) pendant 48h à 37°C et 5% de CO₂. Les cellules ont ensuite été incubées avec ou sans (condition non traitée) 0,033% d'extrait pendant 48h. Chaque condition a été effectuée en duplicat. Les ARN totaux ont été extraits à l'aide de TriPure Isolation Reagent^{®} selon le protocole préconisé par le fournisseur. Les ADN complémentaires ont été synthétisés et un transcriptome a été réalisé sur puce Affymetrix GeneChip Human Transcriptome Array 2.0. L'analyse bioinformatique des gènes dont l'expression est modulée *a minima* par un facteur 2 a été effectuée avec le logiciel Ingenuity Pathway Analysis (IPA^{®}, QIAGEN).

### Résultats :

L'extrait est capable de diminuer l'expression transcriptionnelle des kallikréines (KLK) KLK5 et KLK7, deux protéases à serine impliquées dans la desquamation cutanée. La famille des KLK comporte 15 isoformes dont les KLK5 et KLK7 qui sont exprimées dans les couches supérieures cutanées¹ et interviennent notamment dans l'homéostasie épidermique². En effet, ces deux enzymes sont capables de dégrader les cornéodesmosomes superficiels qui assurent la cohésion entre les cornéocytes qui composent la couche cornée.

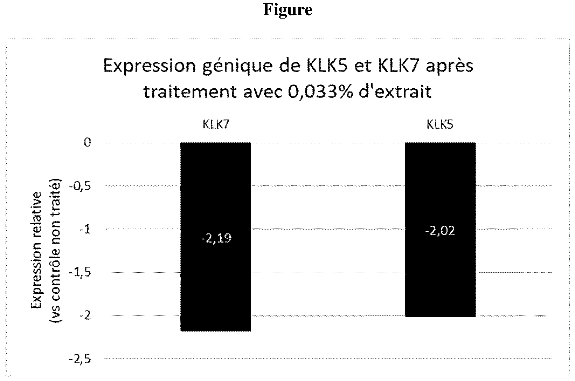

La figure 1 illustre l'inhibition de l'expression transcriptionnelle de KLK5 et KLK7 par l'extrait à 0,033% dans des kératinocytes humains normaux.

### EXEMPLE 2 : Compositions cosmétiques

Les compositions suivantes peuvent être préparées de façon classique pour l'homme du métier. Les quantités indiquées ci-dessous sont exprimées en pourcentages pondéraux. Les ingrédients en majuscules sont identifiés conformément à la dénomination INCI.

### A - lotion après-rasage

| **Nom INCI** | **(% W/W)** |
|---|---|
| Camellia oleifera seed oil | 1-5 |
| Squalane | 1-5 |
| sodium acrylates copolymer & lecithin | 0.1-5 |
| Acrylates/c10-30 alkyl acrylate crosspolymer | 0.1-2 |
| Xanthane gum | 0.01-5 |
| Silica | 0.1-10 |
| Sodium hyaluronate | 0.01-3 |
| Glycerin | 1-30 |
| Alcool | 1-10 |
| Polyquaternium-51 | 1-10 |
| Allantoine | 0.001-5 |
| Extrait selon l'invention | 0.001-10 |
| yeast extract | 0.1-5 |
| Glycols (Caprylyl Glycol and/or Pentylene Glycol and/or Butylene Glycol and/or propanediol) | 0, 1-10 |
| Water | Qs 100 |

### b - gel après-rasage

| **Nom INCI** | **(% w/w)** |
|---|---|
| Lauroyl lysine | 1-5 |
| Limnanthes alba (meadowfoam) seed oil | 1-10 |
| Butyrospermum parkii butter (LIPEX SHEASOFT) | 1-10 |
| Butyrospernum parkii butter extract (LIPEX SHEA TRIS) | 1-10 |
| Camellia oleifera seed oil | 1-10 |
| Extrait selon l'invention | 0.001-10 |
| Squalane | 1-5 |
| Hydrogenated lecithin & glycine soja (soybean) sterols | 1-5 |
| Ammonium acryloyldimethyltaurate/VP copolymer | 1-7 |
| Xanthan gum | 0, 01-2 |
| Agar | 0.1-5 |
| Yeast extract | 1-3 |
| Saccharide isomerate | 1-5 |
| Adenosine | 0.1-0.5 |
| Niacinamide | 0.1-5 |
| Water | Qs 100 |

### c - Gel douche

| **Nom INCI** | **(% w/w)** |
|---|---|
| Sodium methyl cocoyl taurate | 1-15 |
| Sodium methyl oleoyl taurate | 1-10 |
| Sodium cocoyl isethionate | 1-15 |
| Sodium lauroyl sarcosinate | 1-10 |
| Cocamidopropyl betaine | 1-7 |
| Sodium lauroamphoacetate | 1-5 |
| Extrait selon l'invention | 0.001-10 |
| Xanthan Gum | 0, 01-2 |
| Yeast extract | 1-3 |
| Saccharide isomerate | 1-5 |
| Water | Qs 100 |

Ces compositions peuvent être appliquées tous les jours, matin et/ou soir, sur la peau. Elles permettent d'éviter une desquamation excessive et apportent du confort pour l'utilisateur.

## Revendications

1. Extrait alcoolique d'écorces de *Citrus depressa,* comprenant un mélange d'hespéridine, de nobilétine et d'isosinensétine.

2. Extrait alcoolique selon la revendication 1, **caractérisé en ce qu'**il comprend :
- 0.1 à 5% en poids, par rapport au poids d'extrait sec, d'hespéridine, de préférence de 0.5 à 2% en poids,
- 0.1 à 5% en poids, de nobilétine, de préférence de 1 à 2% en poids,
- 0.1 à 3% en poids, d'isosinensétine, de préférence de 0.5 à 1% en poids,
les pourcentages étant exprimés en poids, par rapport au poids d'extrait sec.

3. Extrait alcoolique selon la revendication 1 ou 2, **caractérisé en ce que** l'hespéridine, la nobilétine et l'isosinensétine sont présent en un rapport pondéral hespéridine/nobilétine/isosinensétine compris entre 0.5/1/1.5 et 1.5/3/4 et de préférence, est de 1.1/1.4/0.7.

4. Composition cosmétique ou dermatologique comprenant, dans un véhicule cosmétiquement ou pharmaceutiquement acceptable, un extrait alcoolique extrait d'écorces de *Citrus depressa* selon l'une quelconque des revendications 1 à 3.

5. Composition cosmétique ou dermatologique selon la revendication 4, **caractérisée en ce qu'**elle est adaptée à une application par voie topique.

6. Procédé d'extraction d'écorces de *Citrus depressa,* comprenant les étapes suivantes :
a) extraction des écorces de *Citrus depressa,* préalablement séchées et broyées, avec au moins un solvant alcoolique ;
b) filtration du mélange issu de l'étape a) et optionnellement, décoloration du filtrat obtenu par adsorption sur charbon actif,
c) concentration de l'extrait sec par évaporation jusqu'à une teneur en extrait sec comprise entre 5 et 20%, de préférence entre 9 et 12%,
d) décantation du mélange obtenu en c) pendant au moins 6h jusqu'à obtenir 4 phases distinctes;
e) séparation des phases formées à l'étape d) par élimination des deux phases inférieures solide et huileuse, et de la phase supérieure d'huile essentielle, et récupération de la phase alcoolique comprenant l'extrait d'écorces de *Citrus depressa.*

7. Procédé selon la revendication 6, dans lequel l'étape a) est opérée avec de l'éthanol, à une température comprise entre 50°C et 70°C pendant 1h à 5h.

8. Procédé selon l'une quelconque des revendications 6 ou 7, dans lequel l'extraction est opérée en deux fois :
a) une première extraction des écorces de *Citrus depressa,* préalablement séchées et broyées, avec de l'éthanol, à une température comprise entre 50°C et 70°C pendant 1h à 5h , puis tamisage entre 50 µm et 150µm;
a') une seconde extraction des drèches d'écorces de *Citrus depressa* obtenues à l'étape a), avec de l'éthanol, à une température comprise entre 50°C et 70°C pendant 1h à 5h , puis tamisage entre 50 µm et 150µm.

9. Procédé selon l'une quelconque des revendications 6 à 8, dans lequel l'étape b) de filtration est opérée jusqu'à un seuil de 30 µm, de préférence de 15 µm, et en ce que la décoloration du filtrat sur charbon actif est opérée pendant 1 à 6h, de préférence pendant 3h, puis le charbon est éliminé par filtration jusqu'à un seuil de 5 µm, de préférence de 2µm.

10. Procédé selon l'une quelconque des revendications 6 à 9, comprenant en outre les étapes suivantes :
f) filtration de la fraction alcoolique récupérée à l'étape e) jusqu'à un seuil de 5 µm, de préférence de 2µm puis dilution finale de l'extrait filtré dans un autre solvant alcoolique,
g) évaporation du solvant d'extraction introduit à l'étape a) jusqu'à atteindre une concentration résiduelle en solvant d'extraction inférieure à 1%, de préférence inférieure à 0,5%
h) décantation du mélange obtenu en g) pendant au moins 6h puis filtration jusqu'à un seuil de 5 µm, de préférence de 2µm.

11. Utilisation cosmétique d'un extrait alcoolique d'écorces de *Citrus depressa* selon l'une quelconque des revendications 1 à 3 pour limiter/protéger de la desquamation excessive et contribuer au confort cutané.

## Patentansprüche

1. Alkoholischer Extrakt aus *Citrus* depressa-Schalen, umfassend ein Gemisch von Hesperidin, Nobiletin und Isosinensetin.

2. Alkoholischer Extrakt nach Anspruch 1, **dadurch gekennzeichnet, dass** er Folgendes umfasst:
- 0,1 bis 5 Gew.-% Hesperidin, bezogen auf das Gewicht des Trockenextrakts, vorzugsweise 0,5 bis 2 Gew.-%,
- 0,1 bis 5 Gew.-% Nobiletin, vorzugsweise 1 bis 2 Gew.-%,
- 0,1 bis 3 Gew.-% Isosinensetin, vorzugsweise 0,5 bis 1 Gew.-%,
wobei die Prozentangaben als Gewicht, bezogen auf das Gewicht des Trockenextrakts, ausgedrückt sind.

3. Alkoholischer Extrakt nach Anspruch 1 oder 2, **dadurch gekennzeichnet, dass** Hesperidin, Nobiletin und Isosinensetin in einem Gewichtsverhältnis von Hesperidin/Nobiletin/Isosinensetin vorliegen, das zwischen 0,5/1/1,5 und 1,5/3/4 liegt und vorzugsweise 1,1/1,4/0,7 ist.

4. Kosmetische oder dermatologische Zusammensetzung, umfassend, in einem kosmetisch oder pharmazeutisch akzeptablen Vehikel, einen alkoholischen Extrakt aus *Citrus* depressa-Schalen nach einem der Ansprüche 1 bis 3.

5. Kosmetische oder dermatologische Zusammensetzung nach Anspruch 4, **dadurch gekennzeichnet, dass** sie an die topische Anwendung angepasst ist.

6. Verfahren zur Extraktion von *Citrus depressa-*Schalen, umfassend die folgenden Schritte:
a) Extraktion der zuvor getrockneten und gemahlenen *Citrus* depressa-Schalen mit mindestens einem alkoholischen Lösungsmittel,
b) Filtration des Gemischs aus Schritt a) und gegebenenfalls Entfärbung des erhaltenen Filtrats durch Adsorption an Aktivkohle,
c) Aufkonzentration des Trockenextrakts durch Verdampfung bis auf einen Gehalt an Trockenextrakt zwischen 5 und 20 %, vorzugsweise zwischen 9 und 12 %,
d) Dekantierung des unter c) erhaltenen Gemischs während mindestens 6 Std., bis 4 getrennte Phasen erhalten werden,
e) Trennung der in Schritt d) gebildeten Phasen durch Beseitigung der beiden unteren, festen und öligen Phasen und der oberen Phase des ätherischen Öls und Gewinnung der alkoholischen Phase, die den Extrakt von *Citrus* depressa-Schalen enthält.

7. Verfahren nach Anspruch 6, wobei Schritt a) mit Ethanol bei einer Temperatur zwischen 50 °C und 70 °C während 1 Std. bis 5 Std. durchgeführt wird.

8. Verfahren nach einem der Ansprüche 6 oder 7, **dadurch gekennzeichnet, dass** die Extraktion in zwei Schritten durchgeführt wird:
a) einer ersten Extraktion der zuvor getrockneten und gemahlenen *Citrus* depressa-Schalen mit Ethanol bei einer Temperatur zwischen 50 °C und 70 °C während 1 Std. bis 5 Std. und anschließender Siebung zwischen 50 µm und 150 µm,
a') einer zweiten Extraktion des in Schritt a) erhaltenen Trebers von *Citrus* depressa-Schalen mit Ethanol bei einer Temperatur zwischen 50 °C und 70 °C während 1 Std. bis 5 Std. und anschließender Siebung zwischen 50 µm und 150 µm.

9. Verfahren nach einem der Ansprüche 6 bis 8, wobei der Filtrationsschritt b) bis zu einem Schwellenwert von 30 µm, vorzugsweise 15 µm, durchgeführt wird, und wobei die Entfärbung des Filtrats über Aktivkohle während 1 bis 6 Std., vorzugsweise während 3 Std., durchgeführt wird und dann die Kohle durch Filtration bis zu einem Schwellenwert von 5 µm, vorzugsweise 2 µm, entfernt wird.

10. Verfahren nach einem der Ansprüche 6 bis 9, das ferner die folgenden Schritte umfasst:
f) Filtration der in Schritt e) gewonnenen alkoholischen Fraktion bis zu einem Schwellenwert von 5 µm, vorzugsweise 2 µm, und anschließende endgültige Verdünnung des filtrierten Extrakts in einem anderen alkoholischen Lösungsmittel,
g) Verdampfen des in Schritt a) eingebrachten Extraktionslösungsmittels, bis eine Restkonzentration an Extraktionslösungsmittel von weniger als 1 %, vorzugsweise weniger als 0,5 %, erreicht ist,
h) Dekantierung des unter g) erhaltenen Gemischs während mindestens 6 Std. und anschließende Filtration bis zu einem Schwellenwert von 5 µm, vorzugsweise 2 µm.

11. Kosmetische Verwendung eines alkoholischen Extrakts von *Citrus* depressa-Schalen nach einem der Ansprüche 1 bis 3, um übermäßige Desquamation einzuschränken/davor zu schützen und zum Wohlbefinden der Haut beizutragen.

## Claims

1. Alcoholic extract of *Citrus depressa* peel, comprising a mixture of hesperidin, nobiletin and isosinensetin.

2. Alcoholic extract according to Claim 1, **characterized in that** it comprises:
- 0.1% to 5% by weight, based on the weight of dry extract, of hesperidin, preferably from 0.5% to 2% by weight,
- 0.1% to 5% by weight of nobiletin, preferably from 1% to 2% by weight,
- 0.1% to 3% by weight of isosinensetin, preferably from 0.5% to 1% by weight,
the percentages being expressed by weight based on the weight of dry extract.

3. Alcoholic extract according to Claim 1 or 2, **characterized in that** hesperidin, nobiletin and isosinensetin are present in a hesperidin/nobiletin/isosinensetin weight ratio that is between 0.5/1/1.5 and 1.5/3/4 and is preferably 1.1/1.4/0.7.

4. Cosmetic or dermatological composition comprising, in a cosmetically or pharmaceutically acceptable vehicle, an alcoholic extract of *Citrus depressa* peel according to any one of Claims 1 to 3.

5. Cosmetic or dermatological composition according to Claim 4, **characterized in that** it is suitable for topical application.

6. Method for extracting *Citrus depressa* peel, comprising the following steps:
a) extracting previously dried and ground *Citrus depressa* peel with at least one alcoholic solvent;
b) filtering the mixture obtained from step a) and optionally decolourizing the resulting filtrate by adsorption on activated carbon,
c) concentrating the dry extract by evaporating down to a dry extract content of between 5 and 20%, preferably between 9 and 12%,
d) settling the mixture obtained in c) for at least 6 h until 4 distinct phases are obtained;
e) separating the phases formed in step d) by removing the two solid and oily lower phases and the essential oil upper phase and recovering the alcoholic phase comprising the extract of *Citrus depressa* peel.

7. Method according to Claim 6, wherein step a) is carried out with ethanol at a temperature of between 50°C and 70°C for 1 h to 5 h.

8. Method according to either one of Claims 6 and 7, wherein the extraction is carried out in two stages:
a) performing a first extraction of the previously dried and ground *Citrus depressa* peel with ethanol at a temperature of between 50°C and 70°C for 1 h to 5 h, and then screening between 50 µm and 150 µm;
a') performing a second extraction of the solid *Citrus depressa* peel material obtained in step a) with ethanol at a temperature of between 50°C and 70°C for 1 h to 5 h, and then screening between 50 µm and 150 µm.

9. Method according to any one of Claims 6 to 8, wherein the filtering step b) is carried out down to a cutoff of 30 µm, preferably 15 µm, and wherein the filtrate is decolourized on activated carbon for 1 to 6 h, preferably for 3 h, after which the charcoal is removed by filtering down to a cutoff of 5 µm, preferably 2 µm.

10. Method according to any one of Claims 6 to 9, further comprising the following steps:
f) filtering the alcoholic fraction recovered in step e) down to a cutoff of 5 µm, preferably 2 µm, followed by a final dilution of the filtered extract in another alcoholic solvent,
g) evaporating the extraction solvent introduced in step a) until a residual concentration of the extraction solvent of less than 1%, preferably less than 0.5%, is reached,
h) settling the mixture obtained in g) for at least 6 h and then filtering down to a cutoff of 5 µm, preferably 2 µm.

11. Cosmetic use of an alcoholic extract of *Citrus depressa* peel according to any one of Claims 1 to 3 for limiting/protecting against excessive desquamation and contributing to skin comfort.
